# EUROPEAN PATENT SPECIFICATION

(11) **EP 3 283 483 B1**
(45) Date of publication and mention of the grant of the patent: **19.12.2018**
(21) Application number: 16717596.7
(22) Date of filing: 13.04.2016
(51) Int. Cl.: C07D 405/06

(54) **CRYSTALLINE ELIGLUSTAT HYDROCHLORIDE**
KRISTALLINES ELIGLUSTATHYDROCHLORID
CHLORHYDRATE D'ÉLIGLUSTAT CRISTALLIN

(30) Priority: 14.04.2015 EP 15163534
(43) Date of publication of application: 21.02.2018
(73) Proprietor: Sandoz AG, 4056 Basel (CH)
(72) Inventor: GALBRAITH, Jana, Craigavon BT63 5QD (GB); MCGEE, Laura, Craigavon BT63 5QD (GB)
(74) Representative: Kluschanzoff, Harald
(86) International application number: PCT/EP2016/058136
(87) International publication number: WO 2016/166170

(56) References cited:
- WO-A1-03/008399
- WO-A1-2011/066352

## Description

### FIELD OF INDUSTRIAL APPLICABILITY

The present disclosure generally relates to crystalline eliglustat hydrochloride. The present disclosure also generally relates to a pharmaceutical composition comprising crystalline eliglustat hydrochloride, as well as crystalline eliglustat hydrochloride for use in the treatment of *Gaucher disease,* and methods for obtaining such forms.

### BACKGROUND OF THE DISCLOSURE

The accumulation of glycosphingolipids has been linked to a number of diseases. Compounds which interfere with the synthesis of glycosphingolipids, for example by inhibiting the enzyme UDP-glucose:N-acyl-sphingosine glucosyltransferase (GlcCer synthase), can lower glycosphingolipid concentrations and thus counteract the detrimental accumulation of glycosphingolipids in so called storage diseases. Eliglustat, the compound shown below as compound of formula (I), is a GlcCer synthase inhibitor which has recently been approced for the treatment of Gaucher disease.

WO 2011/066352 discloses that a salt of eliglustat and tartaric acid having a 2:1 stoichiometry (2 molecules of the monobasic eliglustat per molecule of fully deprotonated tartaric acid) can be crystallized to provide a non-hygroscopic crystalline form. Example 1 discloses that many acids have either failed to produce solid salts with eliglustat or have produced solid forms that were non-crystalline and hygroscopic and therefore unacceptable for use in a pharmaceutical product.

There is thus a need for useful alternatives to the salt of eliglustat and tartaric acid having a 2:1 stoichiometry disclosed in WO 2011/066352.

Different salts and solid state forms (including solvated forms) of an active pharmaceutical ingredient often possess different properties. Different properties of different salts and solid state forms and solvates in turn can allow improved formulations, for example, formulations with improved dissolution profile, or with improved stability and shelf-life. Also processing or handling of the active pharmaceutical ingredient during the formulation process may be improved. Even bioavailability of the drug in the pharmaceutical dosage form may be improved.

A new salt of an active pharmaceutical ingredient may also give rise to new polymorphs or crystalline forms of the new salt which may result in an even further improved pharmaceutical dosage form.

Thus, new salts, solid state forms and solvates of an active pharmaceutical ingredient can have desirable processing properties. They can be easier to handle, better suited for storage, allow better purification or allow better conversion to even further salts or polymorphic forms, compared to previously known salts, solid state forms or solvates.

### SUMMARY OF THE DISCLOSURE

The present disclosure provides crystalline eliglustat hydrochloride, processes for preparing crystalline eliglustat hydrochloride, and pharmaceutical compositions comprising crystalline eliglustat hydrochloride.

The present disclosure also relates to the use of crystalline eliglustat hydrochloride disclosed herein for the preparation of a medicament, preferably for the treatment of Gaucher disease.

The present invention further provides a pharmaceutical composition comprising crystalline eliglustat hydrochloride of the present disclosure and at least one pharmaceutically acceptable excipient.

The present invention also provides crystalline eliglustat hydrochloride for use in the treatment of Gaucher disease, the treatment comprising administering a therapeutically effective amount of crystalline eliglustat hydrochloride of the present disclosure, or at least one of the above pharmaceutical compositions to a person suffering from Gaucher disease.

### BRIEF DESCRIPTION OF THE DRAWINGS

FIG. 1. illustrates the x-ray powder diffraction pattern (XRPD) of crystalline eliglustat hydrochloride when measured at 22°C and using Cu Kα radiation.
FIG. 2. illustrates the thermogravimetric differential thermal analysis (TG-DTA) thermogram of crystalline eliglustat hydrochloride.

### DETAILED DESCRIPTION OF THE DISCLOSURE

WO 2011/066352 states in example 1 that the salt between eliglustat and tartaric acid having a 2:1 stoichiometry readily crystallizes and alleges that this is in contrast to other salts, like the 1:1 salt of eliglustat with hydrochloric acid. WO 2011/066352 discloses a salt between eliglustat and hydrochloric acid that is described to be "hygroscopic and non-crystalline and therefore unacceptable for use in a pharmaceutical product".

In contrast, the present disclosure relates to crystalline forms of eliglustat hydrochloride, which are crystalline and non-hygroscopic and suitable for use in a pharmaceutical product. Crystalline eliglustat hydrochloride is described and characterized herein.

The present disclosure provides crystalline eliglustat hydrochloride that have advantageous properties over other solid state forms of eliglustat and over the hygroscopic and non-crystalline eliglustat hydrochloride of WO 2011/066352, the properties being selected from chemical purity, flowability, solubility, dissolution rate, crystal morphology, polymorphic stability, thermal stability, mechanical stability, storage stability, a low content of residual solvent, a lower degree of hygroscopicity, flowability, and advantageous processing and handling characteristics such as compressibility and bulk density.

### Definitions

As used herein "polymorph" refers to crystalline forms having the same chemical composition but different spatial arrangements of the molecules, atoms, and/or ions forming the crystal.

As used herein "solvate" refers to a crystalline form of a molecule, atom, and/or ions that further comprises molecules of a solvent or solvents incorporated into the crystalline lattice structure. The solvent molecules in the solvate may be present in a regular arrangement and/or a non-ordered arrangement. The solvate may comprise either a stoichiometric or nonstoichiometric amount of the solvent molecules. For example, a solvate with a nonstoichiometric amount of solvent molecules may result from partial loss of solvent from the solvate. When the solvent is water, the solvate is often referred to as a "hydrate". When the solvent is present in stoichiometric amount, the solvate may be referred to by adding greek numeral prefixes. For example, a hydrate may be referred to as monohydrate, dihydrate, tri- hydrate etc., depending on the water / eliglustat stoichiometry. The solvent content can be measured, for example, by GC, 'H-NMR or Karl-Fischer (KF) titration.

As used herein "amorphous" refers to a solid form of a molecule, atom, and/or ions that is not crystalline. An amorphous solid does not display a definitive X-ray diffraction pattern.

As used herein, the term "substantially pure" with reference to a particular polymorphic form means that the polymorphic form includes less than 10%, preferably less than 5%, more preferably less than 3%, most preferably less than 1% by weight of any other physical forms of the compound.

The term "free form" refers to the compound per se without salt formation or association with a solvent (e.g., solvate).

The term "peak" used herein corresponds to its typical meaning in the art of XRPD. It may generally mean peaks clearly visible in XRPD patterns. Further, a "peak" may, in a corresponding XRPD pattern, resemble a single peak at a given specified position, or it may represent a double-peak or multiple peaks around a given specified (central) position of the denoted "peak".

The term "essentially the same" with reference to X-ray powder diffraction peak positions means that typical peak position and intensity variability are taken into account. For example, one skilled in the art will appreciate that the peak positions (2θ) will show some inter-apparatus variability, typically as much as 0.2°. Further, one skilled in the art will appreciate that relative peak intensities will show inter-apparatus variability as well as variability due to degree of crystallinity, preferred orientation, prepared sample surface, and other factors known to those skilled in the art, and should be taken as qualitative measure only.

Crystalline eliglustat hydrochloride may be referred to herein as being characterized by graphical data "as shown in" a Figure. Such data include, for example, powder X-ray difractograms (XRPD), differential scanning calorimetry (DSC) thermograms and thermogravimetric analysis (TGA) . The person skilled in the art understands that factors such as variations in instrument type, response and variations in sample directionality, sample concentration and sample purity may lead to small variations for such data when presented in graphical form, for example variations relating to the exact peak positions and intensities.

However, a comparison of the graphical data in the Figures herein with the graphical data generated for an unknown solid form and the confirmation that two sets of graphical data relate to the same crystal form is well within the knowledge of a person skilled in the art.

"Anhydrous" as used herein refers to a solid form which contains not more than 1% (w/w) of water, the water content being determined according to Karl Fischer coulometric titration.

"Room temperature" as used herein means a temperature of from 18°C to 25°C, such as 22°C.

"Reduced pressure" as used herein means a pressure of from 10 mbar to 100 mbar.

Various analytical methods may be used for characterization.

### I. X-ray Powder Diffraction Measurements (XRPD)

One of ordinary skill in the art will appreciate that an X-ray diffraction pattern may be obtained with a measurement error that is dependent upon the measurement conditions employed. In particular, it is generally known that intensities in a X-ray diffraction pattern may fluctuate depending upon measurement conditions employed. It should be further understood that relative intensities may also vary depending upon experimental conditions and, accordingly, the exact order of intensity should not be taken into account. Additionally, a measurement error of diffraction angle for a conventional X-ray diffraction pattern is typically about 5% or less, and such degree of measurement error should be taken into account as pertaining to the aforementioned diffraction angles. Consequently, it is to be understood that the crystal forms of the instant invention are not limited to the crystal forms that provide X-ray diffraction patterns completely identical to the X-ray diffraction patterns depicted in the accompanying Figures disclosed herein. Any crystal forms that provide X-ray diffraction patterns substantially identical to those disclosed in the accompanying Figures fall within the scope of the present invention. The ability to ascertain substantial identities of X-ray diffraction patterns is within the purview of one of ordinary skill in the art.

### II. Simultaneous application of thermogravimetry (TG) and differential thermal analysis (DTA) or "Thermogravimetric Differential Thermal Analysis" (TG/DTA)

TG-DTA generally refers to the simultaneous application of thermogravimetry (TG) and differential thermal analysis (DTA) to one and the same sample in a single instrument. The measurement conditions are perfectly identical for the TG and DTA signals (same atmosphere, gas flow rate, vapor pressure of the sample, heating rate, thermal contact to the sample crucible and sensor, radiation effect, etc.). TG can provide information about physical phenomena, such as second-order phase transitions, including vaporization, sublimation, absorption, adsorption, and desorption, while DTA can provide information on the transformations that have occurred, such as glass transitions, crystallization, melting and sublimation.

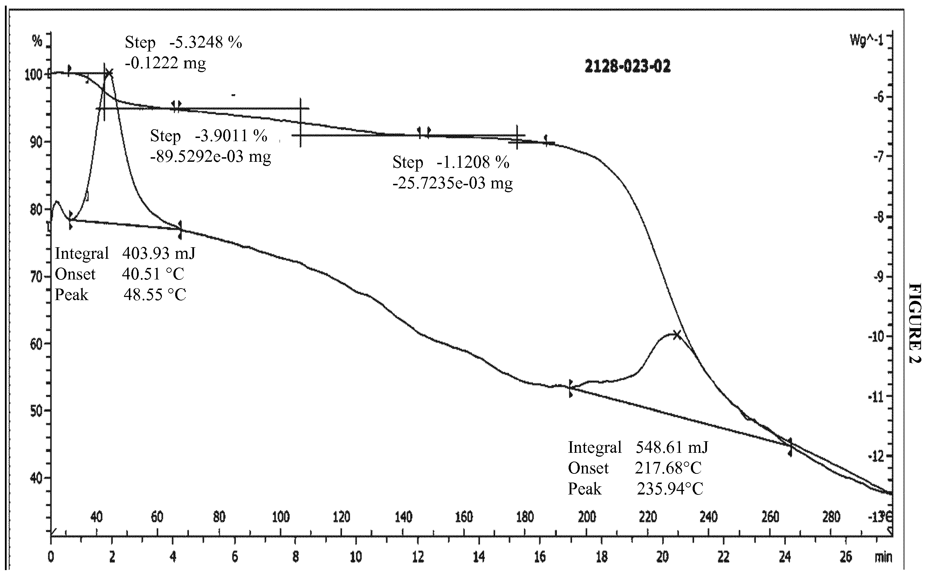

Thermogravimetric analyses were carried out on a Mettler Toledo TGA/DSC1 STARe. The calibration standards were indium and tin. Samples were placed in an aluminium sample pan, inserted into the TG furnace and accurately weighed. A typical sample size was about 20mg, but the exact sample size is not critical. The heat flow signal was stabilised for one minute at 30°C, prior to heating to 300°C in a stream of nitrogen at a rate of 10°C/minute. The skilled person will appreciate that the TG trace is the trace starting at about 100% at 0min in the TG/DTA of figure 2.

The present invention relates to crystalline eliglustat hydrochloride, in particular to a crystalline eliglustat hydrochloride hydrate. Surprisingly it has been found that eliglustat can form a crystalline salt with hydrochloric acid, which salt has properties that make it useful for use in pharmaceutical formulations.

Eliglustat hydrochloride has a molar ratio of eliglustat and chloride typically and preferably in a range of about 1.0 : 0.7 to 1.3, more preferably in a range of about 1.0 : 0.8 to 1.2 , and in particular of about 1.0 : 1.0.

In one aspect the crystalline eliglustat hydrochloride of the present invention may be characterized by a x-ray powder diffraction pattern (XRPD) comprising four or more 2θ values (CuKα λ=1.5418 Å) selected from the group consisting of 6.8, 12.8, 13.7, 15.0, 24.0 and 24.5 measured at a temperature of about 22°C and an x-ray wavelength, λ, of 1.5418 Å. Preferably, the crystalline eliglustat hydrochloride may be characterized by a x-ray powder diffraction pattern comprising five or more 2θ values (CuKα λ=1.5418 Å) selected from the group consisting of 6.8, 12.8, 13.7, 15.0, 24.0 and 24,5 at a temperature of about 22°C.

Preferably, crystalline eliglustat hydrochloride is characterized by a XRPD comprising characteristic peaks at 2-Theta angles of 6.8, 12.8, 13.7, 15.0, 24.0 and 24.5. Crystalline eliglustat hydrochloride may be further characterized by a XRPD further comprising one or more additional characteristic peaks at 2-Theta angles of 13.4 ± 0.2° and 18.5 ± 0.2°.

In one embodiment of the disclosure, crystalline eliglustat hydrochloride is provided in substantially pure form. The crystalline form of eliglustat hydrochloride in substantially pure form may be employed in pharmaceutical compositions which may optionally include one or more other components selected, for example, from the group consisting of excipients, carriers, and one of other active pharmaceutical ingredients active chemical entities of different molecular structure.

Preferably, the crystalline form of eliglustat hydrochloride has substantially pure phase homogeneity as indicated by less than 10%, preferably less than 5 %, and more preferably less than 2 % of the total peak area in the experimentally measured XRPD pattern arising from the extra peaks that are absent from the simulated XRPD pattern. Most preferred is a crystalline form having substantially pure phase homogeneity with less than 1% of the total peak area in the experimentally measured XRPD pattern arising from the extra peaks that are absent from the simulated XRPD pattern.

In one embodiment, a composition is provided consisting essentially of the crystalline form of eliglustat hydrochloride of the present invention. The composition of this embodiment may comprise at least 90 weight % of the crystalline form of eliglustat hydrochloride of the present invention, based on the weight of eliglustat hydrochloride in the composition.

The presence of more than one polymorph in a sample may be determined by techniques such as x-ray powder diffraction (XRPD) or solid state nuclear magnetic resonance spectroscopy. For example, the presence of extra peaks in the comparison of an experimentally measured XRPD pattern with a simulated XRPD pattern may indicate more than one polymorph in the sample. The simulated XRPD may be calculated from single crystal x-ray data. see Smith, D.K., "A FORTRAN Program for Calculating X-Ray Powder Diffraction Patterns," Lawrence Radiation Laboratory, Livermore, California, UCRL-7196 (April 1963) or TOPAS program (Total Pattern Analysis Solution, available through Brucker AXS Inc.).

Crystalline eliglustat hydrochloride of the present invention is preferably provided with the advantageous morphology of elongated rods. Crystalline eliglustat hydrochloride of the present invention is preferably provided as a water soluble crystalline, hydrated form.

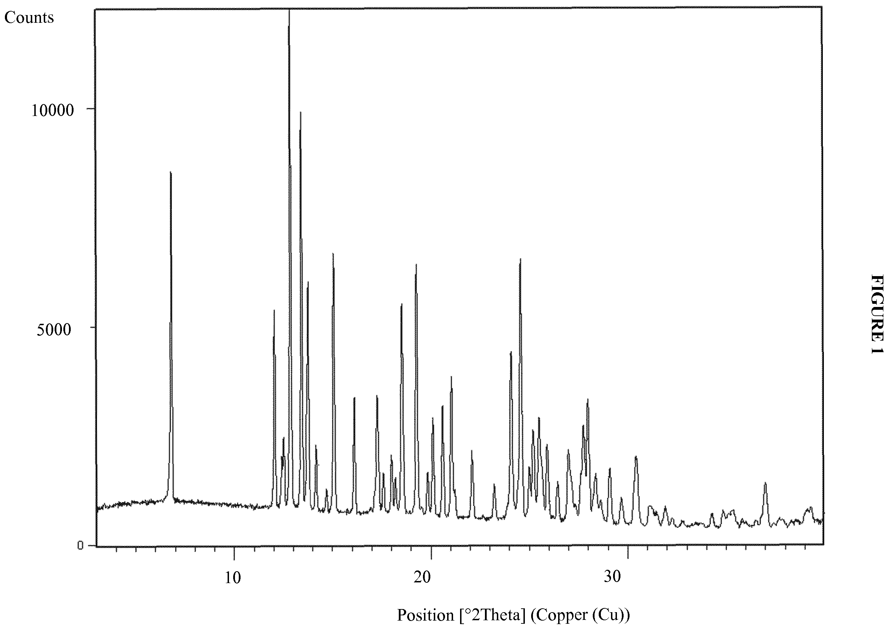

The present invention also provides a crystalline form of eliglustat hydrochloride having a X-ray diffraction spectrum substantially the same as the X-ray powder diffraction spectrum shown in FIG. 1.

Crystalline eliglustat hydrochloride of the present invention, such as crystalline eliglustat hydrochloride hydrate, is stable with regard to its crystallinity even when stored at atmospheric conditions of relatively high relative humidity, for example at 60% relative humidity, at 70% relative humidity or even at 80% relative humidity. The storage temperature under those atmospheric conditions of high relative humidity can be as high as 45°C, such as around 40°C, but the storage temperature can be, of course, lower than 40°C, such as from 0°C to 40°C. This combination of properties facilitates and/or enables the storage and shipment of the active pharmaceutical ingredient in or to countries where the conditions during storage and / or shipment can sometimes not be tightly controlled, such as tropical countries with an Af climate according to the Koppen / Geiger climate classification. This combination of properties further facilitates and / or enables the production of finished dosage forms comprising the crystalline eliglustat hydrochloride of the present invention by processes entailing exposure to high water activity, such as wet granulation. This combination of properties further facilitates and / or enables the production of finished dosage forms comprising the crystalline eliglustat hydrochloride of the present invention intended for use in countries with an Af climate according to the Koppen / Geiger climate classification.

Eliglustat, also called (1R,2R)-octanoic acid[2-(2',3'-dihydro-benzo[1,4]dioxin-6'-yl)-2-hydroxy-1-pyrrolidin-1-ylmethyl-ethyl]-amide, has been approved by the FDA under the brand name Cerdelga TM for the treatment of adult patients with Gaucher disease. It can be produced as eliglustat free form according to the process described in WO03/008399, example p.35. Eliglustat free form is a crystalline solid which starts to melt at about 87°C and which has an XRPD comprising peaks at, for example, 6.0°, 19.0° and 20.8° 2-Theta.

### Preparation of Crystalline Materials:

Crystalline forms may be prepared by a variety of methods, including for example, crystallization or recrystallization from a suitable solvent, sublimation, growth from a melt, solid state transformation from another phase, crystallization from a supercritical fluid, and jet spraying. Techniques for crystallization or recrystallization of crystalline forms from a solvent mixture include, for example, evaporation of the solvent, decreasing the temperature of the solvent mixture, crystal seeding a supersaturated solvent mixture of the molecule and/or salt, freeze drying the solvent mixture, and addition of antisolvents (countersolvents) to the solvent mixture.

Crystals of drugs, including polymorphs, methods of preparation, and characterization of drug crystals are discussed in Solid-State Chemistry of Drugs, S.R. Byrn, R.R. Pfeiffer, and J.G. Stowell, 2nd Edition, SSCI, West Lafayette, Indiana (1999).

For crystallization techniques that employ solvent, the choice of solvent or solvents is typically dependent upon one or more factors, such as solubility of the compound, crystallization technique, and vapor pressure of the solvent. Combinations of solvents may be employed, for example, the compound may be solubilized into a first solvent to afford a solution, followed by the addition of an antisolvent to decrease the solubility of the compound in the solution and to afford the formation of crystals. An antisolvent is a solvent in which the compound has low solubility.

Seed crystals may be added to any crystallization mixture to promote crystallization. Seeding may be employed to control growth of a particular polymorph or to control the particle size distribution of the crystalline product. Accordingly, calculation of the amount of seeds needed depends on the size of the seed available and the desired size of an average product particle as described, for example, in "Programmed Cooling of Batch Crystallizers," J.W. Mullin and J. Nyvlt, Chemical Engineering Science, 1971,26, 369-377. In general, seeds of small size are needed to control effectively the growth of crystals in the batch. Seed of small size may be generated by sieving, milling, or micronizing of large crystals, or by micro-crystallization of solutions. Care should be taken that milling or micronizing of crystals does not result in any change in crystallinity form the desired crystal form (i.e., change to amorphous or to another polymorph).

A cooled crystallization mixture may be filtered under vacuum, and the isolated solids may be washed with a suitable solvent, such as cold recrystallization solvent, and dried under a nitrogen purge to afford the desired crystalline form. The isolated solids may be analyzed by a suitable spectroscopic or analytical technique, such as solid state nuclear magnetic resonance, differential scanning calorimetry, x-ray powder diffraction, or the like, to assure formation of the preferred crystalline form of the product. The resulting crystalline form is typically produced in an amount of greater than about 70 weight % isolated yield, preferably greater than 90 weight % isolated yield, based on the weight of the compound originally employed in the crystallization procedure. The product may be comilled or passed through a mesh screen to delump the product, if necessary.

Preferred processes for the preparation of the crystalline forms of eliglustat hydrochloride of the invention are provided in the examples and in the embodiment section.

Crystalline eliglustat hydrochloride of the invention is useful in the treatment of Gaucher disease. Crystalline eliglustat hydrochloride of the invention may be used alone or in combination, or formulated with one or more excipients or other active pharmaceutical ingredients to provide formulations suitable for the treatment of Gauchers disease identified above. Suitable formulations for eliglustat hydrochloride of the present invention include those described on pages 23 to 26 of WO2015/009927 A1 with the proviso that whenever WO2015/009927 A1 refers to "Formula (I) hemitartrate", it should be replaced by crystalline eliglustat hydrochloride of the present invention. An exemplary capsule formulation of crystalline eliglustat hydrochloride of the present invention comprises eliglustat hydrochloride at an amount equivalent to 84 mg of eliglustat free base and microcrystalline cellulose, lactose monohydrate, hypromellose and glyceryl behenate as excipients.

### Embodiment section

Aspects, advantageous features and preferred embodiments of the present invention are summarized in the following items:
1. Crystalline eliglustat hydrochloride.
2. The crystalline eliglustat hydrochloride according to item 1, which is a hydrate.
3. The crystalline eliglustat hydrochloride according to any one of items 1 or 2 in pure form.
4. The crystalline eliglustat hydrochloride according to any one of aspects 1 to 3 having a molar ratio of eliglustat : chloride in a range of from 1.0 : 0.7 to 1.0 : 1.3
5. The crystalline eliglustat hydrochloride according to item 4, having a molar ratio of eliglustat : chloride in a range of from 1.0 : 0.8 to 1.0 : 1.2.
6. The crystalline eliglustat hydrochloride according to item 5 having a molar ratio of eliglustat : chloride of about 1.0 : 1.0.
7. The crystalline eliglustat hydrochloride according to any one of items 1 to 6 characterized by a x-ray powder diffraction pattern comprising four or more 2θ values selected from the group consisting of 6.8±0.2°, 12.8±0.2°, 13.7±0.2°, 15.0±0.2° and 24.0±0.2° when measured at a temperature of about 22°C using Cu Kα radiation.
8. The crystalline eliglustat hydrochloride according to item 7, characterized by a XRPD comprising characteristic peaks at 2-Theta angles of 6.8±0.2°, 12.8±0.2°, 13.7±0.2°, 15.0±0.2°, 24.0±0.2° and 24.5±0.2°
9. The crystalline eliglustat hydrochloride according to any one of items 7 or 8, wherein the XRPD further comprises at least one additional peak at 2-Theta angles of 13.4 ± 0.2° and 18.5 ± 0.2°, and preferably comprises both peaks.
10. The crystalline eliglustat hydrochloride according to any one of items 7 or 8, wherein the XRPD comprises peaks at all positions identified in list 1.
11. The crystalline eliglustat hydrochloride according to item 10, the X-ray powder diffraction spectrum shown in FIG. 1.
12. The crystalline eliglustat hydrochloride according to any one of items 1 to 11 having a melting and/or decomposition temperature between 190°C and 205°C.
13. The crystalline eliglustat hydrochloride according to any one of items 1 to 12 further characterized by a differential thermal analysis (DTA) thermogram comprising an endotherm peak between 40°C and 60°C.
14. The crystalline eliglustat hydrochloride according to any one of items 1 to 13 further characterized by a DTA thermogram substantially the same as the DTA trace shown in the TG-DTA of FIG. 2.
15. The crystalline eliglustat hydrochloride according to any one of items 1 to 14 further characterized by a thermo gravimetric analysis (TG) trace substantially the same as the TG trace shown in the TG-DTA of FIG. 2.
16. A composition comprising at least 90 weight % of crystalline eliglustat hydrochloride according to any one of items 1 to 15, based the weight of the composition.
17. The composition of item 16 consisting essentially of the crystalline form of any one of items 4 to 15.
18. A pharmaceutical composition comprising crystalline eliglustat hydrochloride according to any one of items 1 to 15 or a composition according to any one of items 16 or 17, and a pharmaceutically acceptable carrier or diluent.
19. The pharmaceutical composition of item 18 which is an oral solid dosage form.
20. The pharmaceutical composition of item 19 which is a capsule.
21. The pharmaceutical composition according to any one of items 19 or 20, further comprising at least one water-soluble filler; at least one water-insoluble filler; at least one binder; and at least one lubricant.
22. Crystalline eliglustat hydrochloride according to any one of items 1 to 15 for use in the treatment of Gaucher disease in a human, the treatment comprising administering to the human a therapeutically-effective amount of crystalline eliglustat hydrochloride according to any one of items 1 to 15.
23. A process of making crystalline eliglustat hydrochloride accoding to any one of items 1 to 15 comprising the step of
   i) allowing eliglustat hydrochloride to crystallize from a solvent comprising a) water and a keton or b) water and a nitrile.
24. The process of item 23, wherein the solvent comprises water and a ketone.
25. The process of item 24, wherein the keton has a solubility in water at 20°C of at least 100g/l.
26. The process of any one of items 24 to 25 wherein the ketone is aceton.
27. The process of any one of items 24 to 26 wherein the solvent comprises at most 5% v/v of a solvent that is not water or a ketone.
28. The process of item 23, wherein the solvent comprises water and a nitrile.
29. The process of item 28, wherein the nitrile has a solubility in water at 20°C of at least 100g/l.
30. The process of any one of items 28 to 29 wherein the nitrile is acetonitrile.
31. The process of any one of items 28 to 30 wherein the solvent comprises at most 5% v/v of a solvent that is not water or a nitrile.
32. The process of any one of items 27 to 31, wherein eliglustat hydrochloride is allowed to crystallize at a temperature of from 0°C to 40°C.
33. The process of any one of items 23 to 32, further comprising the step ii) of exposing the crystalline eliglustat hydrochloride obtained from step i) to an atmosphere of at least 50% and at most 85% relative humidity for storage.
34. The process of item 33, wherein the crystalline eliglustat hydrochloride is stored for at least 7 days under an atmosphere of at least 50% and at most 85% relative humidity.
35. The process of item 33 or item 34, wherein the crystallinity of the crystalline eliglustat hydrochloride is further increased upon storage.

The following non-limiting examples are illustrative of the disclosure and are not to be construed to be limiting to the scope of the claims.

### EXAMPLES

### Preparatory Example

Eliglustat tartrate (stoichiometry eliglustat : tartrate = 2:1) was prepared according to WO 2011/066352. Eliglustat tartrate (5.15g) was stirred in saturated sodium bicarbonate solution (20mL) at room temperature for 30 minutes. Effervescence was observed and the solids dissolved, before precipitating again. The solids were collected by vacuum filtration. The solids were dried under vacuum for 45minutes after which time more solids had precipitated in the initial filtrate. The bulk solids were removed from the filter and the filtrate re-filtered. Solids obtained were added to the bulk material and dried in the vacuum oven at 45°C for 6 hours. The precipitated solid free base material was dried for a further 16hours at room temperature under reduced pressure until constant mass. Eliglustat free base was isolated as a free flowing white powder in ∼54% yield.

Eliglustat free base was crystalline as judged by its XRPD (showing defined peaks) and by its appearance in optical microscopy (birefringent particles). The material was composed of fine needles and irregular shaped particles which tended to aggregate/agglomerate. A DSC thermogram obtained for Eligustat free base displays a single sharp endotherm at an onset of about 87°C. The TG-DTA thermogram showed a melting endotherm at an onset of about 86°C which was in agreement with DSC data. A minimal weight loss of ∼1.6% (equivalent to ∼0.3mol.eq of H2O) was observed between 30°C and 80°C, suggesting the free base to be anhydrous.

### Example 1

Eliglustat free base (3.8g) was stirred in acetonitrile (40mL) at ambient temperature. To the suspension was added 1M HCl solution (9.86mL). On addition of HCl the solids immediately dissolved. The solution was stirred overnight at room temperature. The solution was then transferred to a 100mL beaker and evaporated at room temperature under a flow of N₂. Once solvent levels had decreased to about 15mL a precipitate was observed around the sides of the beaker. The solids were scraped into the solvent and after ≤10 minutes a slurry was obtained which was too thick to stir due to precipitation. The solids were collected by filtration and air dried under reduced pressure for 1.5 hours. XRPD analysis was carried out on these solids prior to drying (about 16hrs) under reduced pressure at room temperature. On drying the material had decreased in weight by about 100mg. The material was placed back to drying under reduced pressure at room temperature for a further 1.5 hours. The material lost a further about 20mg in weight. No further drying was carried out as the solid material obtained was free flowing. Crystalline eliglustat hydrochloride was prepared in ∼78% yield (3.22g).

### Example 2

XRPD analyses were performed on crystalline eliglustat hydrochloride from example 1 using a Panalytical Xpert Pro diffractometer equipped with a Cu X-ray tube and a Pixcel detector system. The isothermal samples were analysed in transmission mode and held between low density polyethylene films. The following XRPD program was used: range 3-40°2θ, step size 0.013°, counting time 99sec, ∼22min run time.

Significant peaks were observed at the following positions [± 0.2 °2Th.] (list 1):
6,8; 12,0; 12,4; 12,8; 13,4; 13,7; 15,0; 16,1; 17,2; 17,6; 17,0; 18,2; 18,5; 19,2; 19,8; 20,1; 20,6; 21,0; 22,0; 23,2; 24,0; 24,5; 25,0; 25,1; 25,4; 25,8; 26,4; 26,9; 27,7; 27,9; 28,3; 29,0; 29,6; 30,4; 31,1; 31,81.

### Example 3. Thermogravimetric Differential Thermal Analysis (TG-DTA)

Thermogravimetric analyses were carried out on crystalline eliglustat hydrochloride from example 1 on a Mettler Toledo TGA/DSC1 STARe. The calibration standards were indium and tin. Samples were placed in an aluminium sample pan, inserted into the TG furnace and accurately weighed. The heat flow signal was stabilised for one minute at 30°C, prior to heating to 270°C in a stream of nitrogen at a rate of 10°C/minute.

The TG/DTA thermogram is displayed in Figure 2. There is a sharp endotherm at peak temperature 45.5°C with an associated weight loss of about 5.3%, which corresponds to about 1.4 molar equivalents (mol. eq.) of water. A further gradual weight loss of about 5.0% was observed between 65-190°C, which corresponds to about 1.3 mol. eq. of water (assuming no loss of HCl). No further endothermic events were observed until the melt onset with decomposition at 195°C.

HCl stoichiometry was assessed by ¹H NMR and Eliglustat hydrochloride was found to be a mono-hydrochloride salt. The analysis suggests Eliglustat hydrochloride from example 1 to be a crystalline hydrate with an onset of melt with decomposition at 195°C.

### Example 4. Solubility

Aqueous solubility was determined gravimetrically by slurrying Eliglustat hydrochloride from example 1 in water at 22°C. The pH of Eliglustat HCl in water was 7.6. A filtered aliquot was transferred to a pre-weighed vial and the solution evaporated. XRPD analysis of the remaining solid eliglustat hydrochloride was consistent with Eliglustat hydrochloride form A. The aqueous solubility of Eliglustat hydrochloride at 22°C was 33g/L.

### Example 5 Stability

Crystalline eliglustat hydrochloride from example 1 was stored for one week at 40°C / 75% relative humidity. XRPD analysis after storage showed that the crystalline eliglustat hydrochloride remained stable with regard to its crystalline form, and the same peaks as listed for example 2 were observed. The material even seemed to have slightly increased in crystallinity during the storage period.

### Example 6. Capsule preparation

Crystalline eliglustat hydrochloride, microcrystalline cellulose, lactose monohydrate, hypromellose and glyceryl behenate are separately passed though a 20 mesh screen. Amounts of the screened ingredients as indicated below are blended in a high shear granulator for 15 min. The ingredients are then processed according to example 10 of WO 2011/066352 A.

## Claims

1. Crystalline eliglustat hydrochloride.

2. The crystalline eliglustat hydrochloride according to claim 1, which is a hydrate.

3. The crystalline eliglustat hydrochloride according to any one of claims 1 or 2 in pure form.

4. The crystalline eliglustat hydrochloride according to any one of claims 1 to 3 **characterized by** a x-ray powder diffraction pattern comprising four or more 2θ values selected from the group consisting of 6.8± 0.2°, 12.8± 0.2°, 13.7± 0.2°, 15.0± 0.2°, 24.0± 0.2° and 24.5± 0.2° when measured at a temperature of about 22°C using Cu Kα radiation.

5. The crystalline eliglustat hydrochloride according to claim 4, having the X-ray powder diffraction spectrum shown in FIG. 1.

6. The crystalline eliglustat hydrochloride according to any one of claims 1 to 5 having a melting and/or decomposition temperature between 190°C and 205°C.

7. The crystalline eliglustat hydrochloride according to any one of claims 1 to 6 **characterized by** having a differential thermal analysis (DTA) thermogram comprising an endotherm peak between 40°C and 60°C.

8. A composition comprising at least 90 weight % of crystalline eliglustat hydrochloride according to any one of claims 1 to 7, based the weight of the composition.

9. The composition of claim 8 consisting essentially of the crystalline form of any one of claims 4 to 7.

10. A pharmaceutical composition comprising crystalline eliglustat hydrochloride according to any one of claims 1 to 7 or a composition according to any one of claims 8 or 9, and a pharmaceutically acceptable carrier or diluent.

11. Crystalline eliglustat hydrochloride according to any one of claims 1 to 7 for use in the treatment of Gaucher disease in a human, the treatment comprising administering to the human a therapeutically-effective amount of crystalline eliglustat hydrochloride according to any one of claims 1 to 7.

12. A process of making crystalline eliglustat hydrochloride comprising the step of allowing eliglustat hydrochloride to crystallize from a solvent comprising a) water and a ketone or b) water and a nitrile.

13. The process of claim 12, wherein the ketone is acetone.

14. The process of claim 12, wherein the nitrile is acetonitrile.

15. The process of any one of claims 12 to 14, wherein eliglustat hydrochloride is allowed to crystallize at a temperature of from 0°C to 40°C.

## Patentansprüche

1. Kristallines Eliglustat-Hydrochlorid.

2. Kristallines Eliglustat-Hydrochlorid nach Anspruch 1, das ein Hydrat ist.

3. Kristallines Eliglustat-Hydrochlorid nach einem der Ansprüche 1 oder 2 in reiner Form.

4. Kristallines Eliglustat-Hydrochlorid nach einem der Ansprüche 1 bis 3, **gekennzeichnet durch** ein Röntgenpulverbeugungsmuster, das vier oder mehr 2θ-Werte umfasst, die aus der Gruppe ausgewählt werden, die aus 6,8 ± 0,2 °, 12,8 ± 0,2 °, 13,7 ± 0,2 °, 15,0 ± 0,2 °, 24,0 ± 0,2 ° und 24,5 ± 0,2 ° besteht, bei Messung bei einer Temperatur von etwa 22 °C mithilfe von Cu Kα-Strahlung.

5. Kristallines Eliglustat-Hydrochlorid nach Anspruch 4, das das Röntgenpulverbeugungsspektrum aufweist, das in FIG. 1 dargestellt ist.

6. Kristallines Eliglustat-Hydrochlorid gemäß einem der Ansprüche 1 bis 5, das eine Schmelz- und/oder Zersetzungstemperatur zwischen 190° C und 205 °C aufweist.

7. Kristallines Eliglustat-Hydrochlorid gemäß einem der Ansprüche 1 bis 6, **dadurch gekennzeichnet, dass** es ein Differenzialthermoanalyse (DTA)-Thermogramm aufweist, das einen endothermen Peak zwischen 40 °C und 60 °C umfasst.

8. Zusammensetzung umfassend mindestens 90 Gew.-% kristallines Eliglustat-Hydrochlorid gemäß einem der Ansprüche 1 bis 7 basierend auf dem Gewicht der Zusammensetzung.

9. Zusammensetzung nach Anspruch 8, die im Wesentlichen aus der kristallinen Form nach einem der Ansprüche 4 bis 7 besteht.

10. Pharmazeutische Zusammensetzung, die kristallines Eliglustat-Hydrochlorid nach einem der Ansprüche 1 bis 7 oder eine Zusammensetzung nach einem der Ansprüche 8 oder 9 und einen pharmazeutisch annehmbaren Träger oder ein pharmazeutisch annehmbares Verdünnungsmittel umfasst.

11. Kristallines Eliglustat-Hydrochlorid nach einem der Ansprüche 1 bis 7 für die Verwendung bei der Behandlung der Gaucher-Krankheit bei einem Menschen, wobei die Behandlung das Verabreichen einer therapeutisch wirksamen Menge von kristallinem Eliglustat-Hydrochlorid nach einem der Ansprüche 1 bis 7 umfasst.

12. Verfahren zum Herstellen von kristallinem Eliglustat-Hydrochlorid, das den Schritt umfasst, dem Eliglustat-Hydrochlorid das Kristallisieren aus einem Lösungsmittel zu erlauben, das aus a) Wasser und einem Keton oder b) Wasser und einem Nitril besteht.

13. Verfahren nach Anspruch 12, wobei das Keton Aceton ist.

14. Verfahren nach Anspruch 12, wobei das Nitril Acetonitril ist.

15. Verfahren nach einem der Ansprüche 12 bis 14, wobei dem Eliglustat-Hydrochlorid das Kristallisieren bei einer Temperatur von 0 °C bis 40 °C erlaubt wird.

## Revendications

1. Chlorhydrate d'éliglustat cristallin.

2. Chlorhydrate d'éliglustat cristallin selon la revendication 1, qui est un hydrate.

3. Chlorhydrate d'éliglustat cristallin selon l'une quelconque des revendications 1 ou 2 sous une forme pure.

4. Chlorhydrate d'éliglustat cristallin selon l'une quelconque des revendications 1 à 3, **caractérisé par** un diagramme de diffraction de rayons X sur poudre comprenant quatre ou plus de valeurs de θ2 choisies dans le groupe constitué de 6,8 ± 0,2°, 12,8 ± 0,2°, 13,7 ± 0,2°, 15,0 ± 0,2°, 24,0 ± 0,2° et 24,5 ± 0,2° lors d'une mesure à une température d'environ 22 °C en utilisant le rayonnement Cu Kα.

5. Chlorhydrate d'éliglustat cristallin selon la revendication 4, ayant le spectre de diffraction de rayons X sur poudre présenté en la FIGURE 1.

6. Chlorhydrate d'éliglustat cristallin selon l'une quelconque des revendications 1 à 5, ayant une température de fusion et/ou de décomposition entre 190 °C et 205 °C.

7. Chlorhydrate d'éliglustat cristallin selon l'une quelconque des revendications 1 à 6, **caractérisé par** un thermogramme d'analyse thermique différentiel (DTA) comprenant un pic endothermique entre 40 °C et 60 °C.

8. Composition comprenant au moins 90 % en poids de chlorhydrate d'éliglustat cristallin selon l'une quelconque des revendications 1 à 7, basés sur le poids de la composition.

9. Composition selon la revendication 8, constituée essentiellement de la forme cristalline selon l'une quelconque des revendications 4 à 7.

10. Composition pharmaceutique comprenant un chlorhydrate d'éliglustat cristallin selon l'une quelconque des revendications 1 à 7 ou composition selon l'une quelconque des revendications 8 ou 9, et un support ou un diluant pharmaceutiquement acceptable.

11. Chlorhydrate d'éliglustat cristallin selon l'une quelconque des revendications 1 à 7, pour une utilisation dans le traitement de la maladie de Gaucher chez un humain, le traitement comprenant l'administration à un humain d'une quantité thérapeutiquement efficace de chlorhydrate d'éliglustat cristallin selon l'une quelconque des revendications 1 à 7.

12. Procédé de fabrication d'un chlorhydrate d'éliglustat cristallin comprenant l'étape de laisser le chlorhydrate d'éliglustat se cristalliser à partir d'un solvant comprenant a) de l'eau et une cétone ou b) de l'eau et un nitrile.

13. Procédé selon la revendication 12, dans lequel la cétone est l'acétone.

14. Procédé selon la revendication 12, dans lequel le nitrile est l'acétonitrile.

15. Procédé selon l'une quelconque des revendications 12 à 14, dans lequel le chlorhydrate d'éliglustat est laissé cristalliser à une température de 0 °C à 40 °C.
